# EUROPEAN PATENT SPECIFICATION

(11) **EP 3 266 369 B1**
(45) Date of publication and mention of the grant of the patent: **22.11.2023**
(21) Application number: 17001157.1
(22) Date of filing: 06.07.2017
(51) Int. Cl.: A61B 5/00, A61B 5/11, G16H 40/67, G16H 80/00, G16H 50/20

(54) **ANALYSIS OF FALL SEVERITY USING A FALL DETECTION SYSTEM AND WEARING APPARATUS**
ANALYSE DER FALLSCHWERE MITTELS EINES FALLDETEKTIONSSYSTEMS UND TRAGEVORRICHTUNG
ANALYSE DE LA GRAVITÉ D'UNE CHUTE PAR UN SYSTÈME DE DÉTECTION DE CHUTE ET APPAREIL PORTATIF

(30) Priority: 06.07.2016 TW 105210160 U
(43) Date of publication of application: 10.01.2018
(73) Proprietor: Leedarson Lighting Co., Ltd., Zhangzhou, Fujian 363999 (CN)
(72) Inventor: CHANG, You-He, Xiamen, Fujian (CN)
(74) Representative: Proi World Intellectual Property GmbH

(56) References cited:
- WO-A1-2010/004538
- WO-A1-2015/091581
- GB-A- 2 323 196
- US-A1- 2008 129 518
- US-A1- 2009 292 227
- US-A1- 2014 276 238
- US-A1- 2016 210 838

## Description

### Technical Field

The present invention relates to a fall detection system and a wearing apparatus, and more particularly to the analysis of fall severity of fall detection system and the wearing apparatus.

### Background of invention

Because of rapid progress of medical hygiene and technology, multiple developed countries have extended average life expectancy of people. The extension of average life expectancy of people is a result of social progress and represents improvement of medical care, hygiene, nutrition, living environment and working conditions and increase of material and wealth. However, relative to the average life expectancy of the countries, same impact on decline in fertility results in changes in demographic structure of the developed countries. Long-term care for elderly disability has become an important subject of public policy. To effectively reduce resources required for human services, and improve the long-term care environment for the elderly are problems the developed countries are going to face in short-term.

For long-term hospitalized patients or elderly people with home care, due to lack of nurses and caregivers, often have alone situation. If the patient or the elderly side without assistance, often have situation of fall and injury. In order to avoid above situation, the current situation is to wear a wearing apparatus with inertial detectors on patients' bodies in many hospitals. Through the inertial detector to detect whether a user falls or not. When the user is detected fall, the message is passed to service center to inform the service center of medical services for corresponding patient or care receivers, so that the service center may send emergency medical personnel to rescue. However, the type of fall detection is often misjudged. Although the fall of the patient or care receiver occurs, the situation is not serious, and still often disturbs the medical staff in the service center, resulting in a large number of medical resources waste, and dispute situation even often occurs.

In order to solve the above problems, the patent application of the People's Republic of China No. 102186420 discloses a fall detection system in which a processor is used for analyzing signals to identify the quiescent period of the user after the fall and comparing the length and the threshold of the user's quiescent period to determine the severity of the fall. The case may determine whether the user is to stand up again, and if the user may stand up again within the threshold time, the user's status is not serious. However, the above discriminant may determine whether the user is in a state of unconsciousness or fainting after falling, but may not exclude the situation that the user is being picked up, or the user is still awake but wounded. Based on the above situation, if only base on the quiescent period of fall and exclude the user needs emergency rescue, may misjudge the serious degree of injury to the user.
US 2014/276238 A1 relates to methods, systems, and apparatuses for detecting fall events of a person. GB 2 323 196 A relates to methods and arrangements for an automatic fall or impact alarm. WO 2015/091581 A1 provided a method of responding to a detected fall. WO 2010/004538 A1 relates to a wearable system and method for monitoring and protecting against falls. US 2008/129518 A1 relates to fall detection system including a wearable monitoring device that monitors the movement of a person; the monitoring device comprises a sensor system including an accelerometer and a communication system; the monitoring device may be configured to detect when a person had arisen and begun to walk after a fall and use this as an indication that the fall was not serious. US 2016/210838 A1 relates to a wearable motion sensing device and system that facilitate monitoring user activity. US 2009/0292227 A1 relates to an apparatus and method for detecting that a patient has fallen.

### Summary of the invention

A main purpose of the invention is to solve the problem that the know-how may not effectively distinguish seriousness of the fall, leading to a misjudged situation.

In order to solve the above problems, the present invention provides, according to independent claim 1, a wearing apparatus for wearing by a user.

The wearing apparatus for wearing by a user comprises an inertial sensor, wherein the inertial sensor is based on the user's motion to obtain user's motion data; a communication module, wherein the communication module is used to link and transmit data to a server for real-time monitoring through the server; and a processor, wherein the processor is connected with the inertial sensor and the communication module. The processor further acquires the user's motion data when the user is to stand up again via the inertial sensor after the processor detects user's fall state based on the user's motion data, matches the user's motion data according to normal posture conditions and / or abnormal posture conditions in a database to report severity of the user's injury to a dispenser via the communication module. The processor is configured to increase the number of times of the inertia sensor drawing the user's motion data per unit time to obtain more accurate data when the processor detects user's fall state. The processor preliminarily records the user's motion data obtained by the user or the normal person's normal posture to establish the normal posture condition.

Therefore, the invention has the following advantages:
The invention may analyze the posture or walking mode of the data to determine the severity of the damage to the user, and decide whether to send emergency ambulance personnel. Compared with the know-how technology, the present invention can more accurately analyze the user's injury and reduce the situation of misjudged of justice to make management be easier to reduce labor costs and to avoid waste of social resources.

The invention may prejudge the seriousness of the user's fall and the situation of the injury by the user's posture or walking mode. The above data may be prepared in advance to prepare the corresponding equipment or the corresponding medical personnel who deal with the injury type to the scene.

### Brief description of the drawings

Fig.1 illustrates a block diagram of a long-term care system.
Fig.2 illustrates a block diagram of a better implemented pattern of an invention.
Fig. 3A, Fig. 3B and Fig. 3C illustrates a data graph of a falling state.
Fig. 4A, Fig. 4B, Fig. 4C and Fig. 4D illustrates a data graph of a normal posture.
Fig. 5A, Fig. 5B and Fig. 5C illustrates the first data graph of an abnormal posture.
Fig. 6A, Fig. 6B and Fig. 6C illustrates the second data graph of an abnormal posture.
Fig. 7A, Fig. 7B, Fig. 7C and Fig. 7D illustrates the third data graph of an abnormal posture.

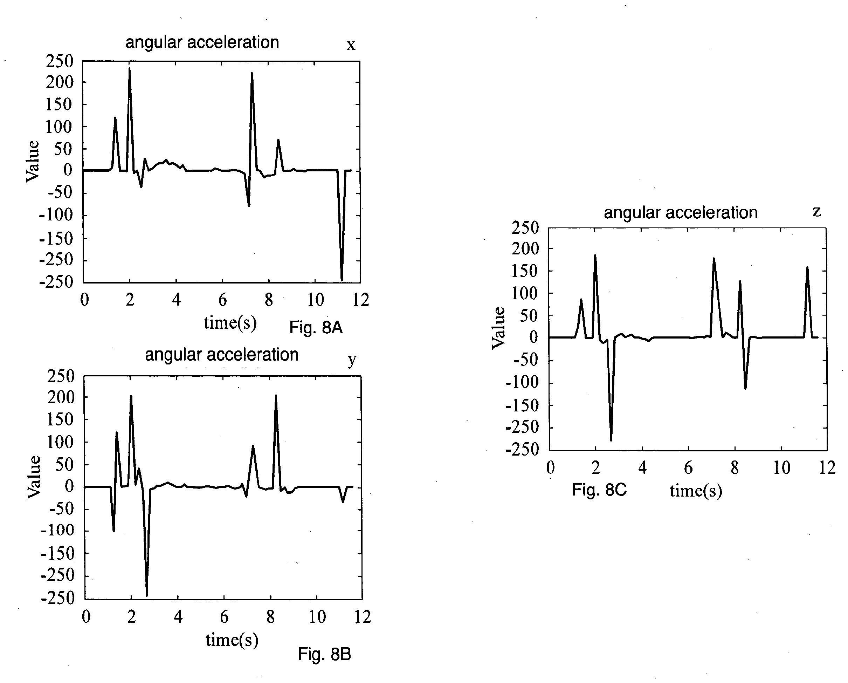

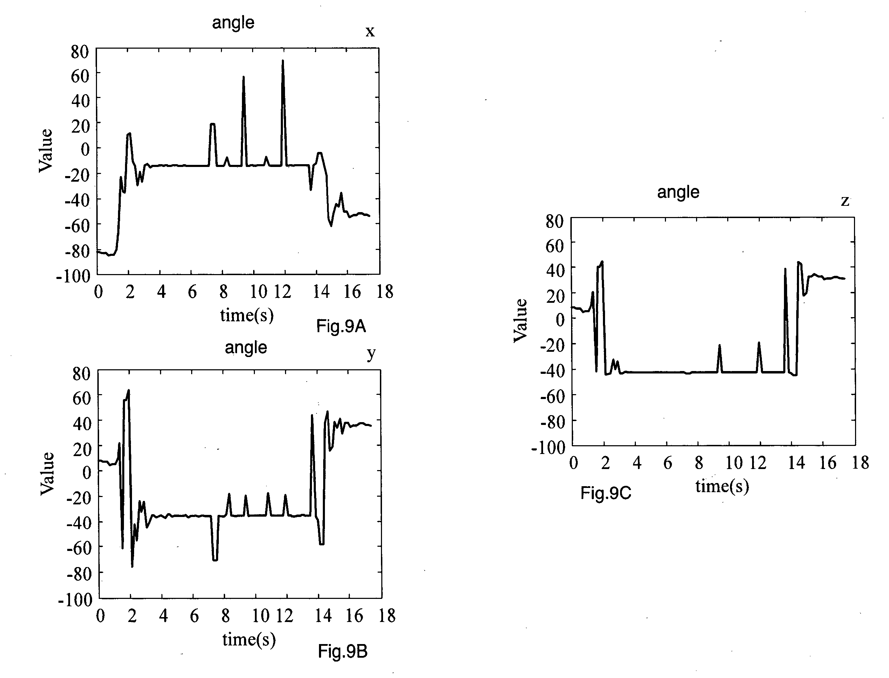

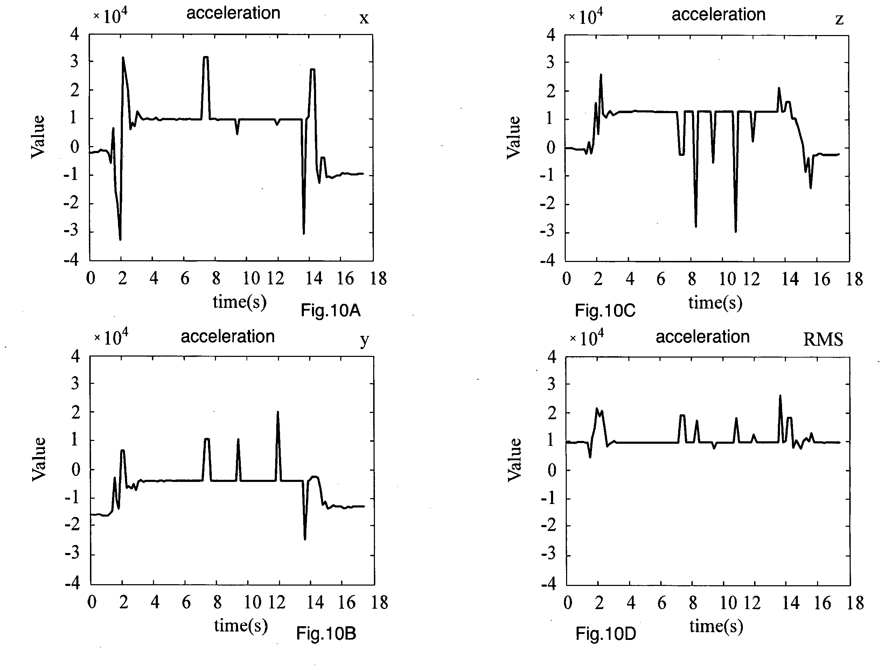

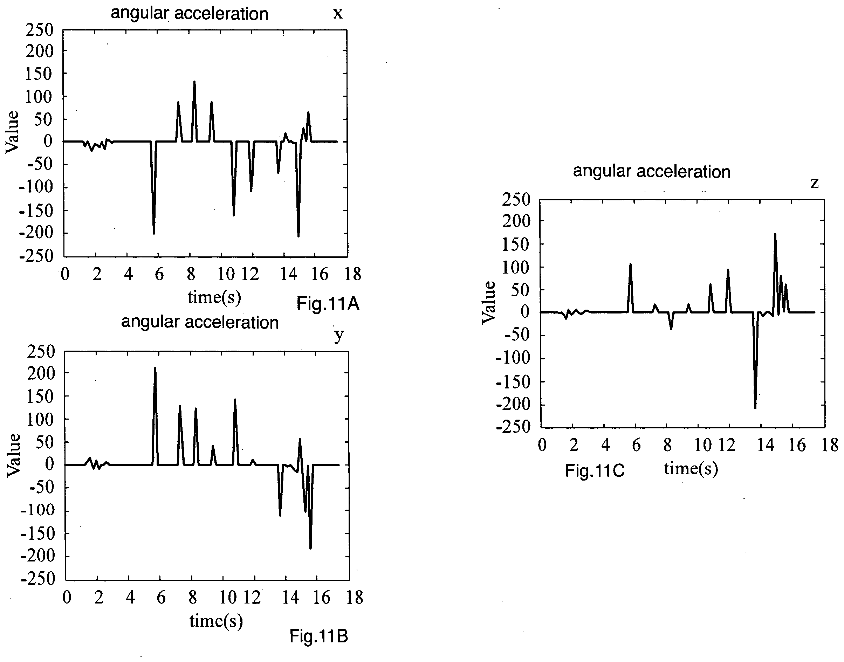

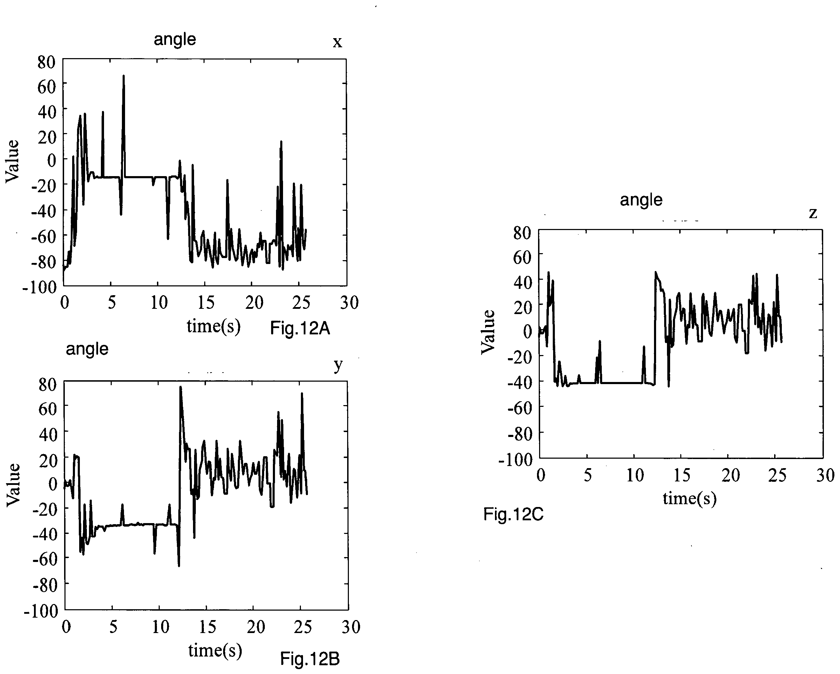

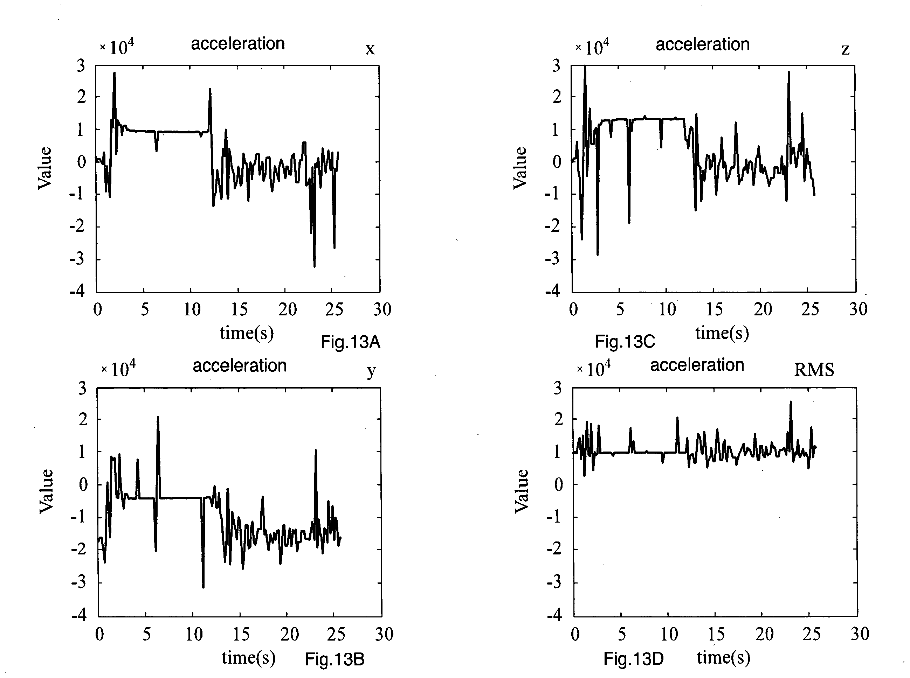

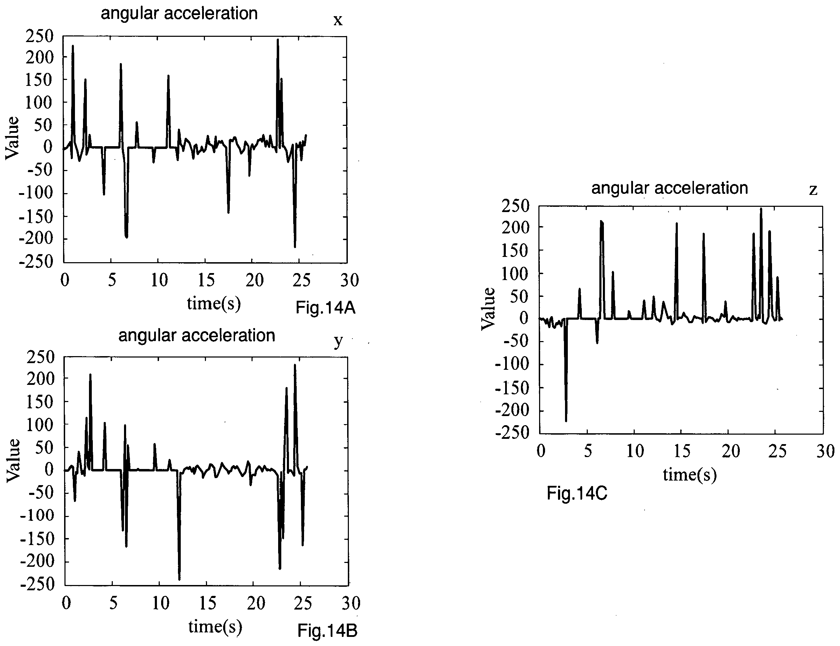

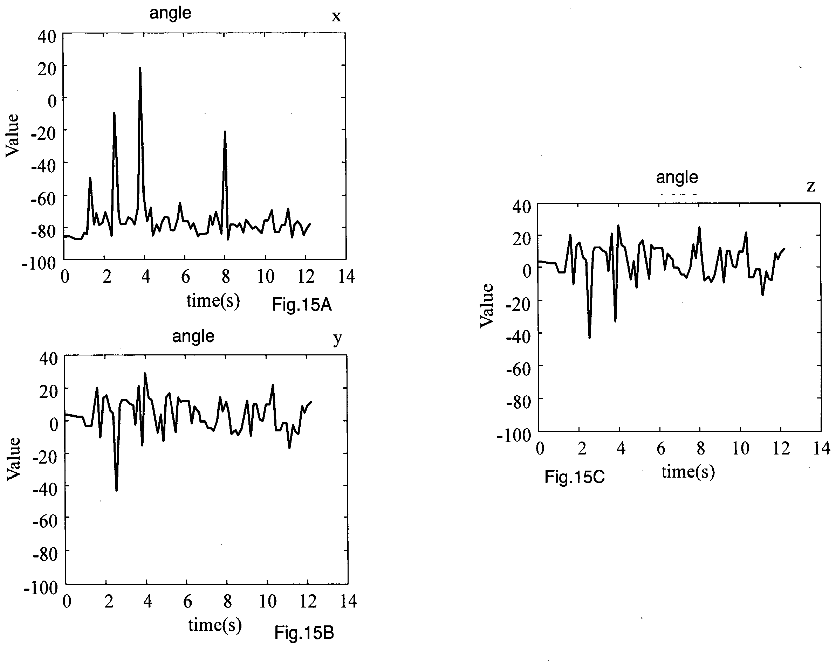

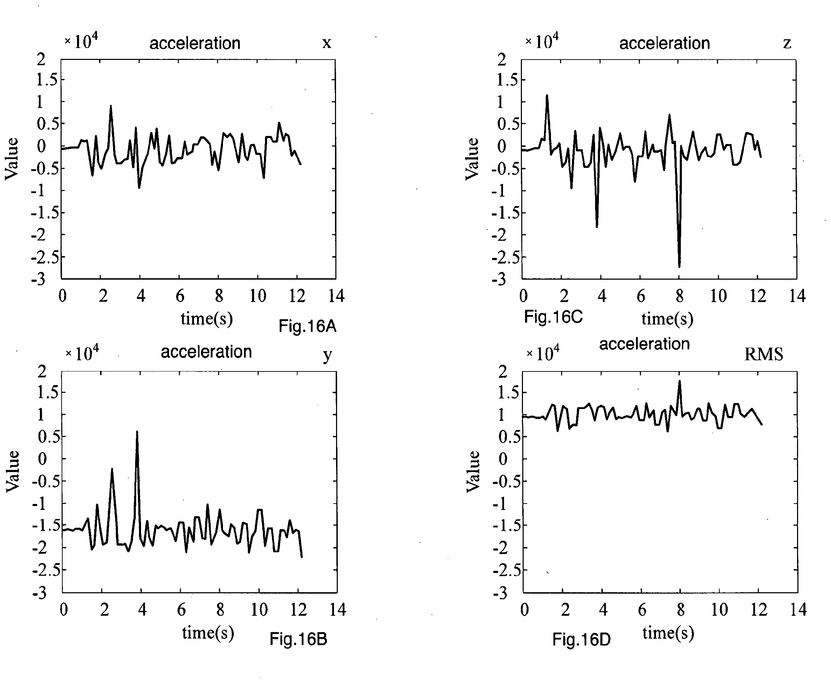

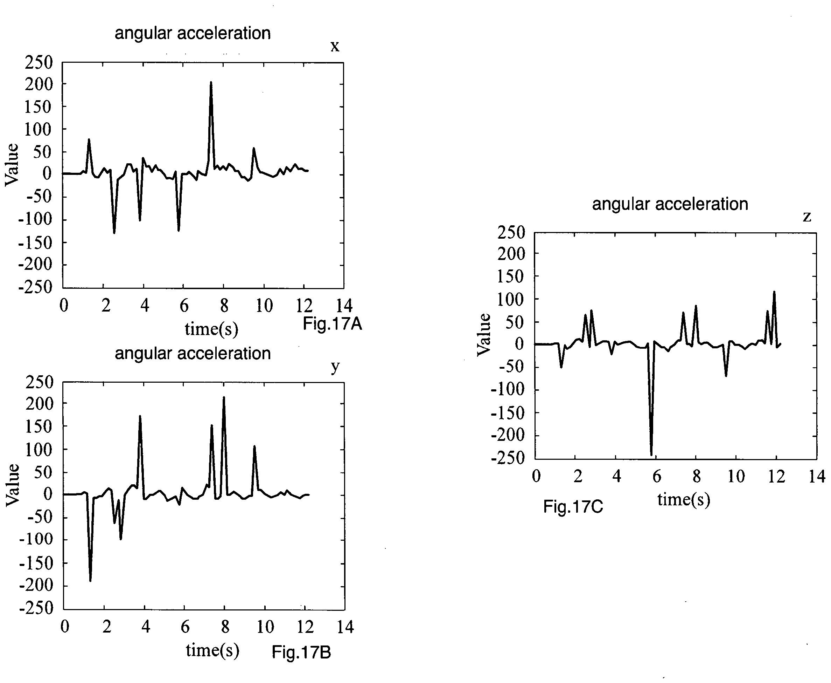

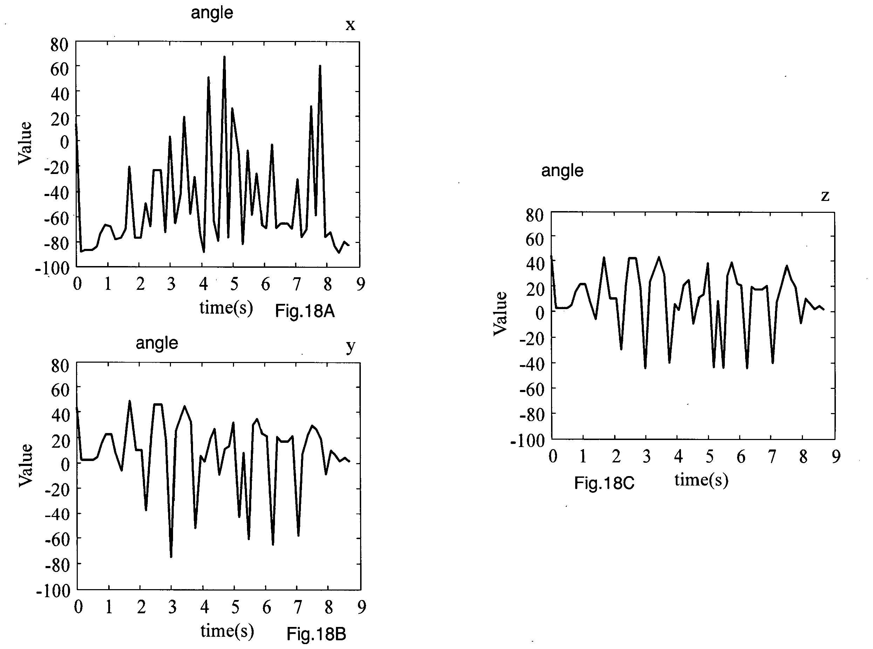

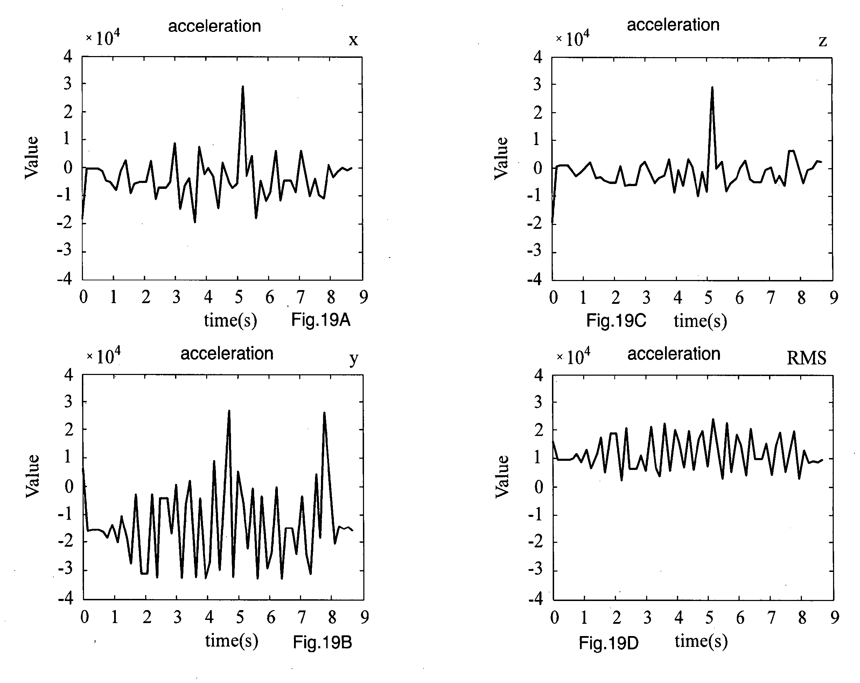

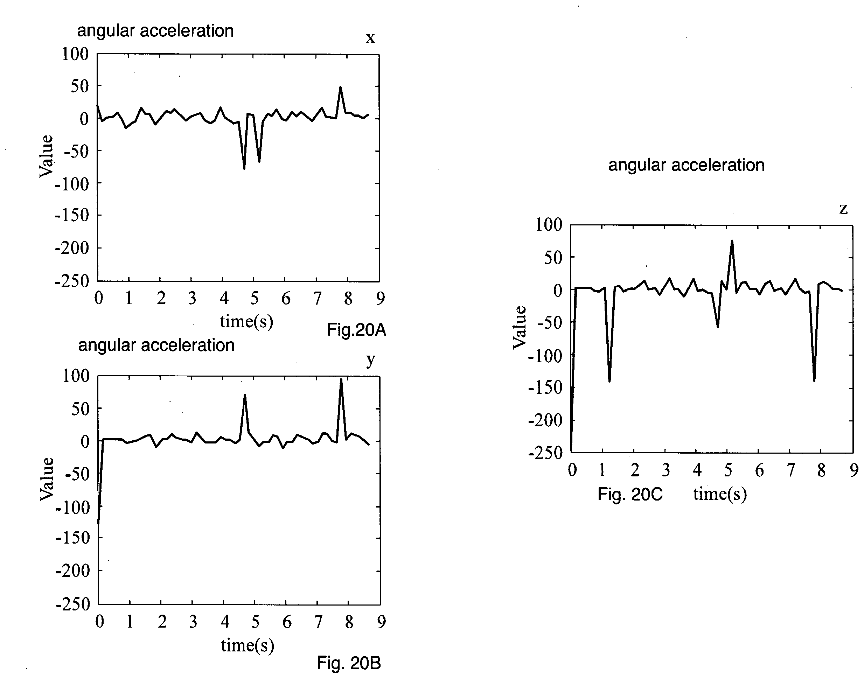

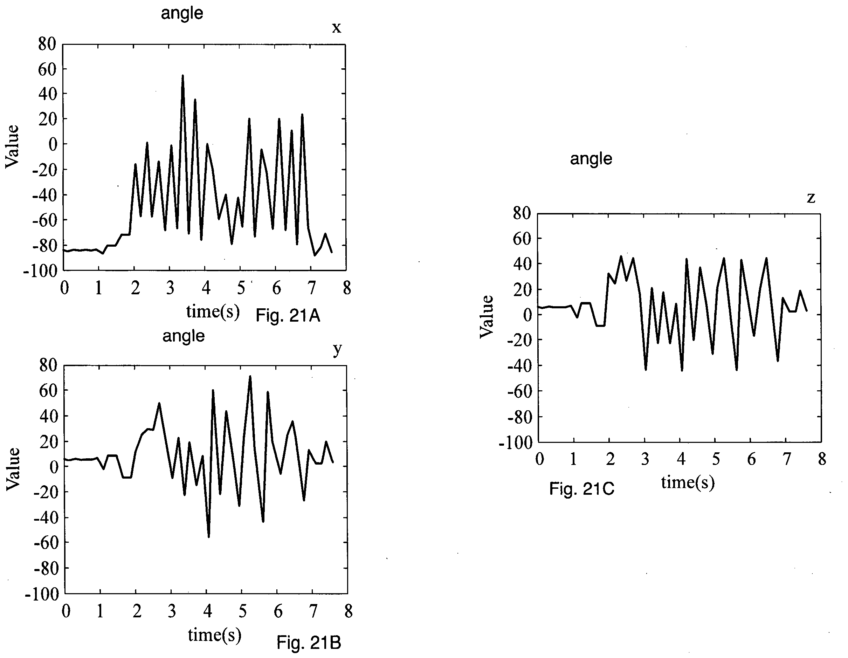

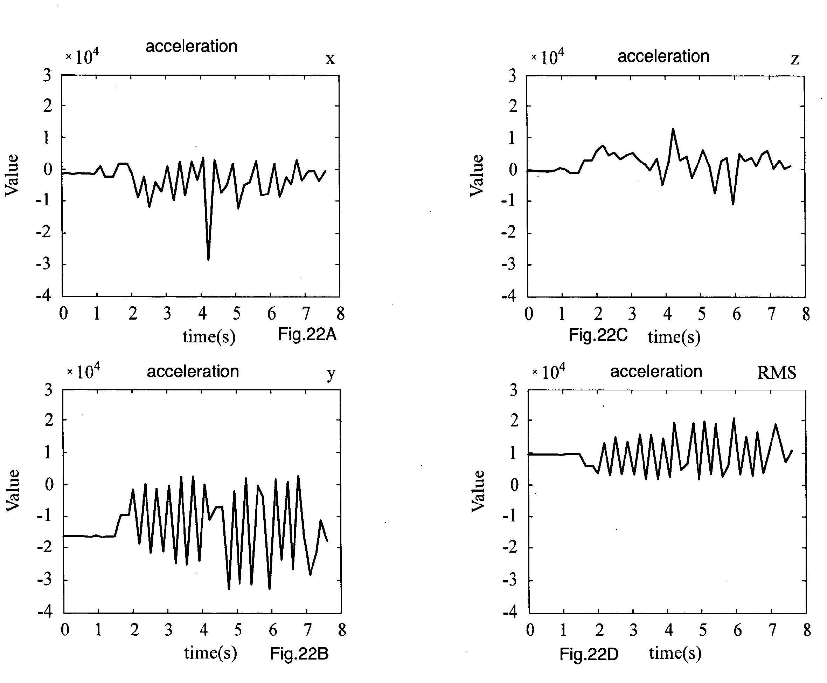

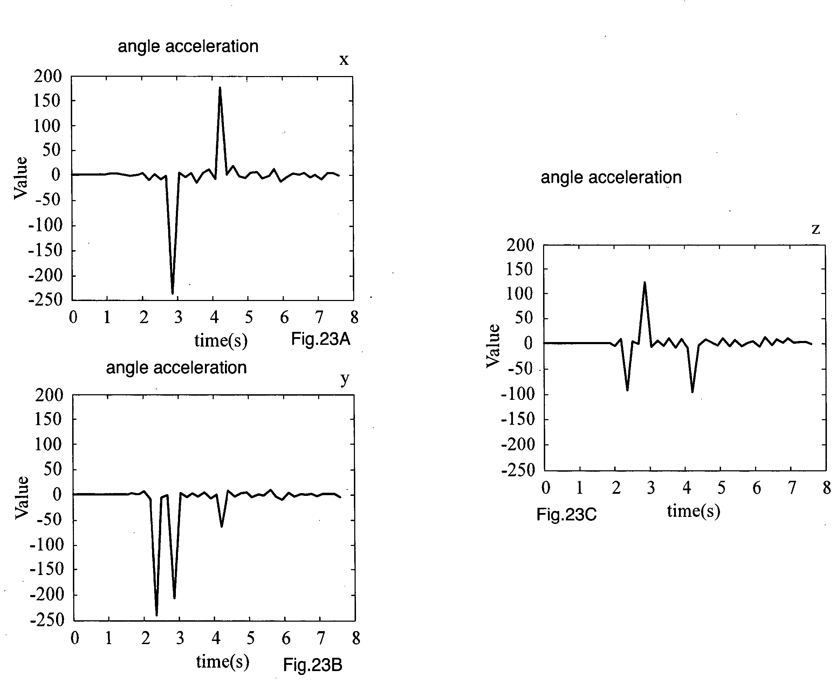

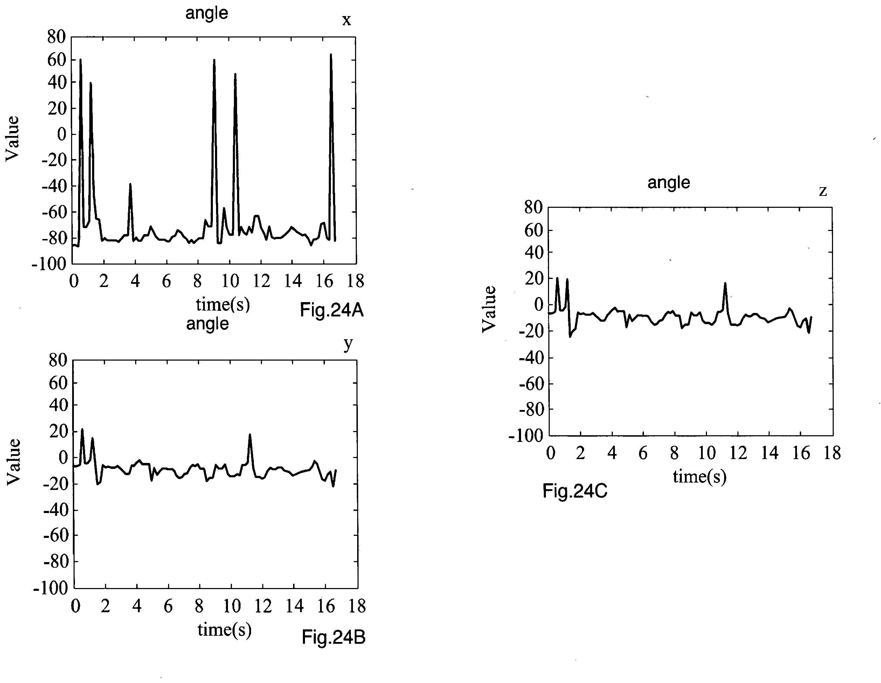

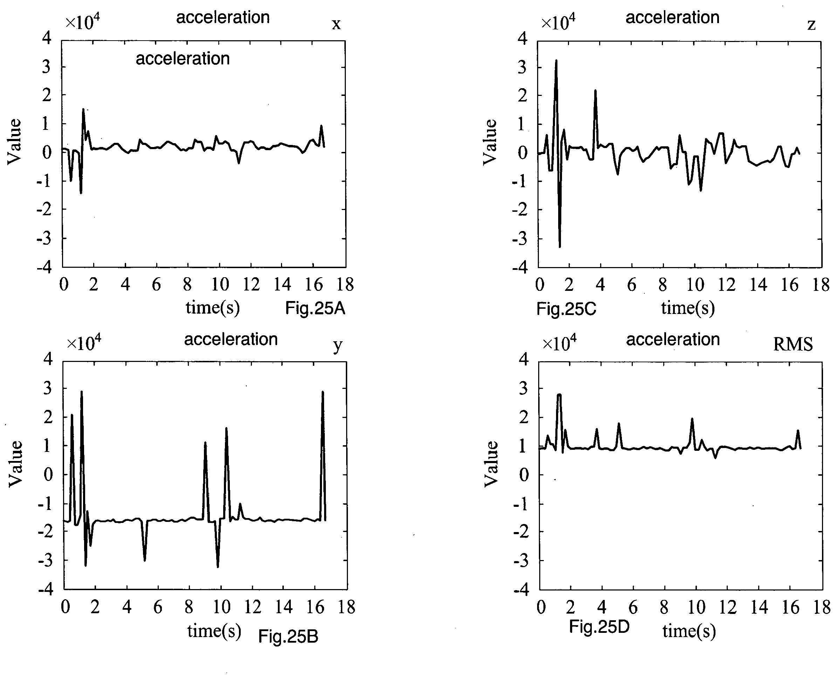

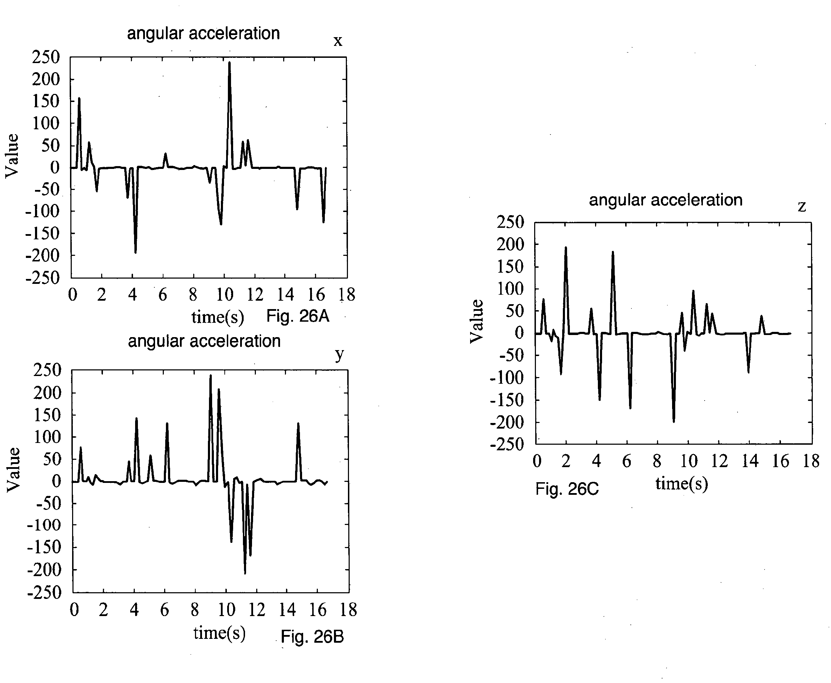

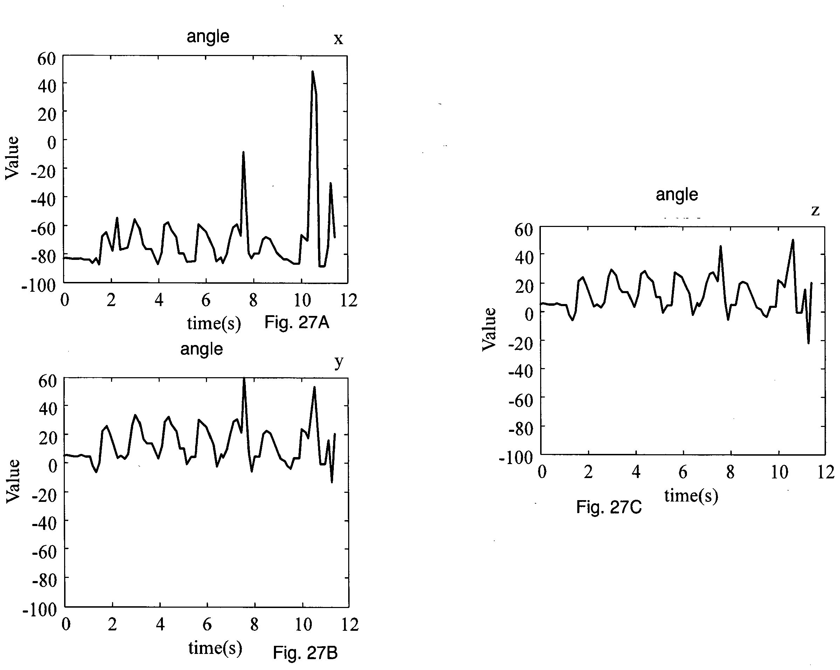

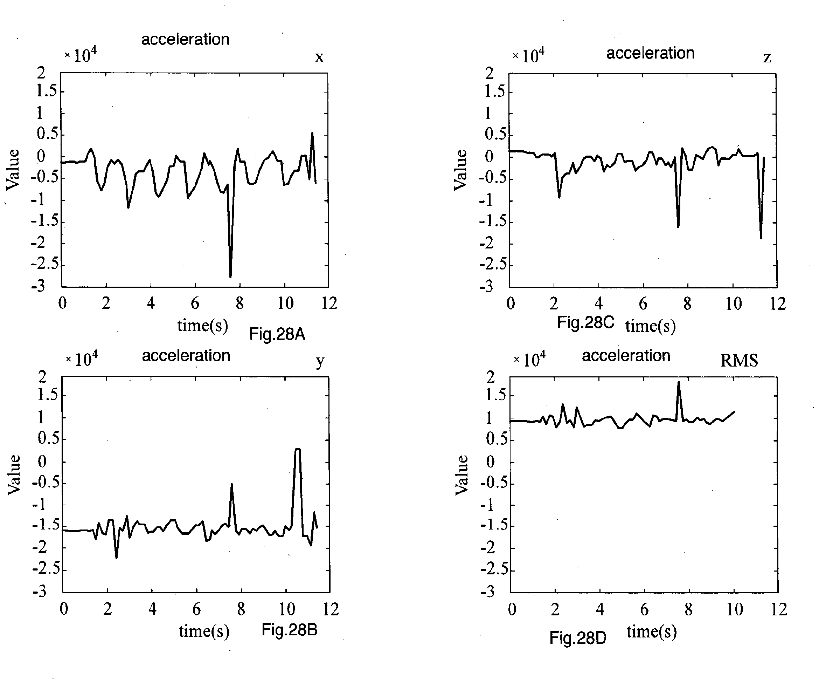

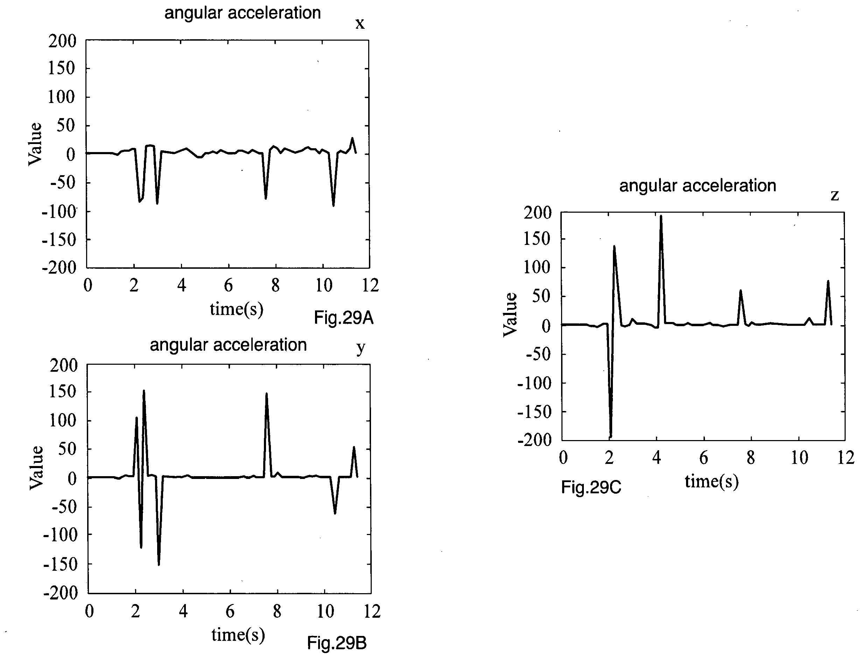

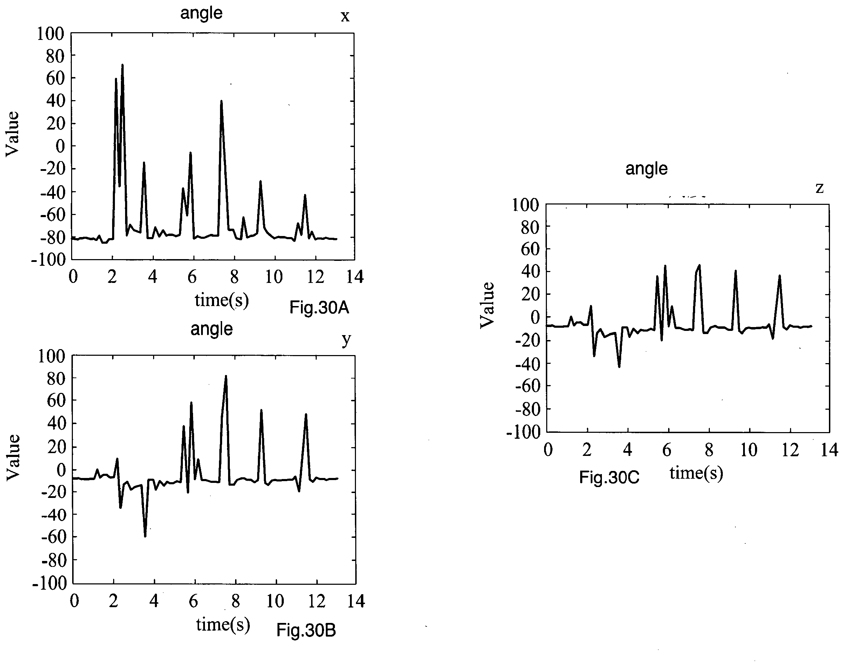

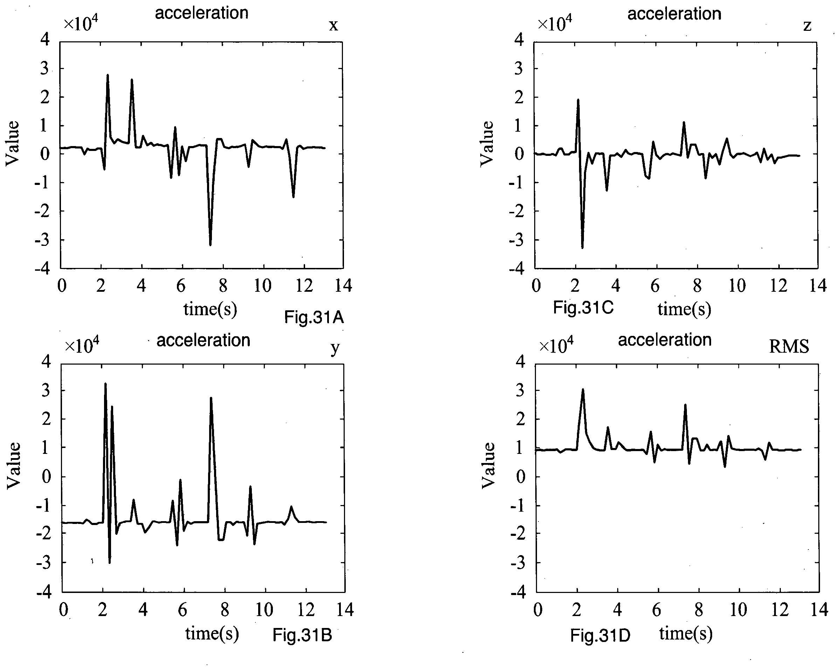

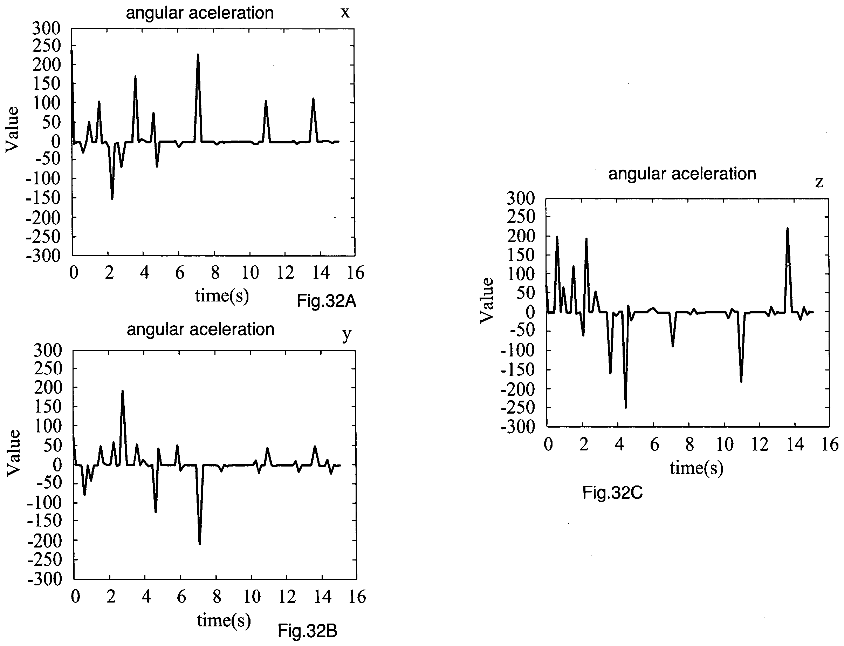

Fig. 8A, Fig. 8B, Fig. 8C, Fig. 9A, Fig. 9B, Fig 9C, Fig. 10A, Fig. 10B, Fig. 10C, Fig.100, Fig. 11A, Fig. 11B, Fig. 11C, Fig. 12A, Fig. 12B, Fig. 12C, Fig. 13A, Fig. 13B,Fig. 13C, Fig. 13D, Fig. 14A, Fig. 14B, Fig. 14C, Fig. 15A, Fig. 15B, Fig. 15C, Fig.16A, Fig. 16B, Fig. 16C, Fig. 16D, Fig. 17A, Fig. 17B, Fig. 17C, Fig. 18A, Fig. 18B,Fig. 18C, Fig. 19A, Fig. 19B, Fig. 19C, Fig. 19D, Fig. 20A, Fig. 20B, Fig. 20C, Fig.21A, Fig. 21B, Fig. 21C, Fig. 22A, Fig. 22B, Fig. 22C, Fig. 22D, Fig. 23A, Fig. 23B,Fig. 23C, Fig. 24A, Fig. 24B, Fig. 24C, Fig. 25A, Fig. 25B, Fig. 25C, Fig. 25D, Fig.26A, Fig. 26B, Fig. 26C, Fig. 27A, Fig. 27B, Fig. 27C, Fig. 28A, Fig. 28B, Fig. 28C,Fig. 28D, Fig. 29A, Fig. 29B, Fig. 29C, Fig. 30A, Fig. 30B, Fig. 30C, Fig. 31A, Fig.31B, Fig. 31C, Fig. 31D, Fig. 32A, Fig. 32B and Fig. 32C illustrate other experiment data.

### Detailed Description

The detailed description and technical contents of the invention are described as following with the Figs.

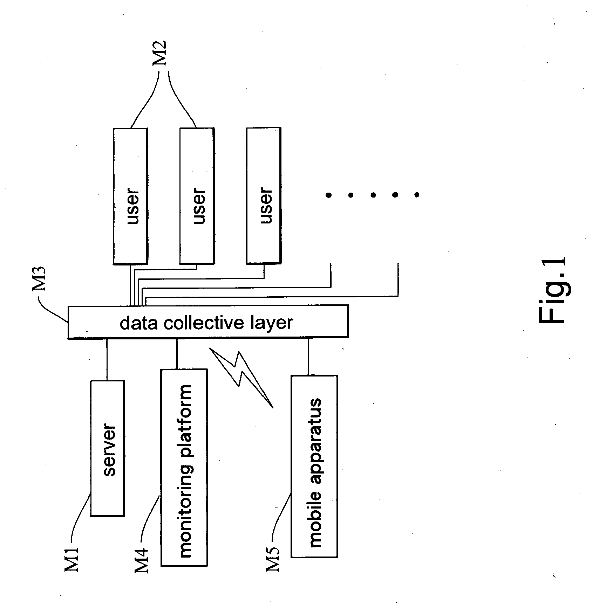

The following is a brief description of the operation of the server, please refer to Fig.1. Fig.1 illustrates a block diagram of a long-term care system. As the Fig shows:
The invention is suitable for a server M1 to perform immediate care for multiple users (care receivers) to perform a remote and immediate care function for a patient or a care receiver.

The server M1 may establish a connection with multiple users M2 via a data collective layer M3, and complete the immediate monitoring of the multiple users M2 by establishing a database dedicated to the user M2. In the real-time data monitoring, the server M1 at the same time based on user motion data to establish a database, and the corresponding data with the user's account to establish relevance. In a preferred embodiment, the wearing apparatus of the user M2 may transmit a data packet containing user fall information to the server M1 via a wireless network. In another preferred embodiment, the wearing apparatus of the user M2 may transmit the user's status (e.g., fall, posture) to the server M1 via a text message.

The server M1 may communicate with a healthcare provider and a monitoring platform M4 via the data collective layer M3 in communication with the multiple users M2 via the data collective layer M3. When the server M1 judges that the user is in need of emergency care, the server M1 transmits emergency message to the monitoring platform M4 and informs the medical staff corresponding to the user may occur.

In addition to the above method, the server M1 may also connect a mobile apparatus M5 to the healthcare providerthrough the data collective layer M3 to transmit the emergency information immediately to the mobile apparatus M5 of the healthcare provider so that the medical staff may do the situation of the user out of the immediate response.

The data collective layer M3 may be one of wireless communication protocols composed by BlueTooth, ZigBee, GPRS, WiFi, GSM, 3G, 4G, HSDPA or WiMax. Through the data collective layer M3 may achieve the possibility of full-time care with limited human resources.

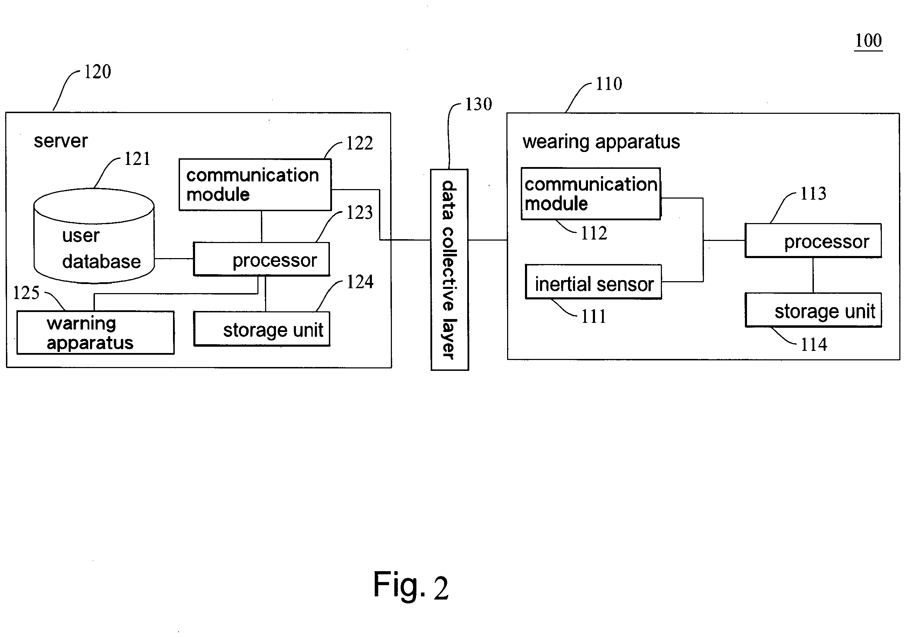

The following description of the invention of the fall detection system, please refer to Fig.2. Fig.2 illustrates a block diagram of a better implemented pattern of an invention. As the Fig shows:
In this embodiment illustrates a fall detection system 100 includes a wearing apparatus 110 with one or more inertial sensors 111, and a server 120 for monitoring user motion data obtained by the one or more inertial sensors 111 in order to achieve real-time monitoring.

The wearing apparatus 110 is for the user to wear. The wearing apparatus 110, for example, may be a spectacle type, a wrist-watch type, a wearing type, a temple type, a sticking type, and etc. The present invention does not limit the detailed description of the wearing apparatus 110.

The wearing apparatus 110 is set with an inertial sensor 111 for detecting user's motion data, a communication module 112, a processor 113 connected to the inertial sensor 111 and the communication module 112, and a storage unit 114 connected to the processor 113. The inertial sensor 111 may be an accelerometer (G-Sensor), magnetizer (M-Sensor), gyroscope (Gyroscope), Gravity sensor (GV-Sensor), linear acceleration sensor (LA-Sensor), rotate vector sensor (RV-Sensor), tilt sensor (Tilt-Sensor) or the assembly of the above apparatus. Through the above apparatus may feel acceleration, angular acceleration, direction, and motion data (hereinafter referred to as the user's motion data). Through the above data may identify whether the user is falling state, and the user's posture.

The server 120 includes a communication module 122, a user database 121, a warning apparatus 125, a processor 123 connected to the communication module 122, the user database 121, and a warning apparatus 125, and a storage unit 124 connected to the processor 123. The communication module 122 is used to communicate with each other via the data layer 130 with one or more wearing apparatuses 110. The user database 121 is used to store user accounts and other related user profiles. The processor 123 is connected to the wearing apparatus 110 via the communication module 122 to access the data of the inertial sensor 111, and the analysis of the user's status, the storage of the data, and the establishment of the user database 121 are performed by the processor 123 of the server 120. The wearing apparatus 110 is only used to optimize the signal obtained by the inertial sensor 111 and to perform data transmission and reception via the communication module 112 and the server 120.

The analysis of the user's status may be performed by the processor 113 at the end of the wearing apparatus 110 and the user's motion data stored in a storage unit 114, thereby increasing the reliability of the identification and reducing the data loss, and the following description may be made based on the present embodiment.

The processor 113, 123, for example, is a central processing unit (CPU) or other programmable microprocessors (microprocessors). The processors 113, 123 are preferably used in conjunction with the storage unit 114 for loading and executing the program in the storage unit 114. The storage unit 114, for example, is of any one type of a fixed or removable random access memory (RAM), a Read-Only Memory (ROM), a flash memory, a hard disk, other similar apparatus, or a combination of the apparatuses, in which a storage unit 114 may connect with the processor 113, 123 to become a processor with a storage unit 114.

The processor 113 is used to load program of the storage unit 114 and perform operations such as fall recognition, posture recognition, and clock adjustment. The processor 113 judges whether the user has fallen based on the user's motion data or not and further detects the user's motion data when the user is on the stand-by again when it is judged that the user's fall state occurs, and determines whether the user's data (maybe stored in the user database 121 or the storage unit 114 of the wearing apparatus 110, which is not limited in the present invention) is compared with the user's motion data to determine the severity of the damage of the user. When the processor 113 detects the abnormal posture of the user, an alert signal is generated based on the type of injury to the warning apparatus 125.

Specifically, the inertial sensor 111 directly provides a three-axis sensing signal, which is an X-axis acceleration (speed) parameter, a Y-axis acceleration (speed) parameter, and a Z-axis acceleration (speed) parameter. In the present embodiment, a three-axis inertial sensor is used for detection. In another preferred embodiment, a six-axis inertial sensor may be used for detection in which a three-axis acceleration is used for detection and the other three-axis angular acceleration is used to verify the reliability of the test results. The invention does not exclude the use of only nine axes, more than nine axes or other similar means to check the user's posture.

The processor 113 may first provide a series of indications for the user to follow the instructions to record the data of the user's normal posture at the beginning of the installation of the wearing apparatus 110 on the user, and the recorded data may be stored in the storage unit 114 (or uploaded to the user database 121 of the server 120) to establish a user-specific normal posture condition.

In another preferred embodiment, the user may wear one or more wearing apparatuses 110 to determine the user's posture and walking mode more clearly at each location of the body by obtaining data corresponding to the respective portions.

For easily explaining and understanding, the X-axis, Y-axis, and Z-axis systems mentioned below correspond to the directions in the real space, where the X-axis and the Y-axis correspond to the right and left sides of the real space, Z-axis corresponds to the vertical direction of the real space, the above relationship is not used to limit the scope of the invention. In addition, some of the figures are plotted with ideal data and are not plotted with actual experimental data.

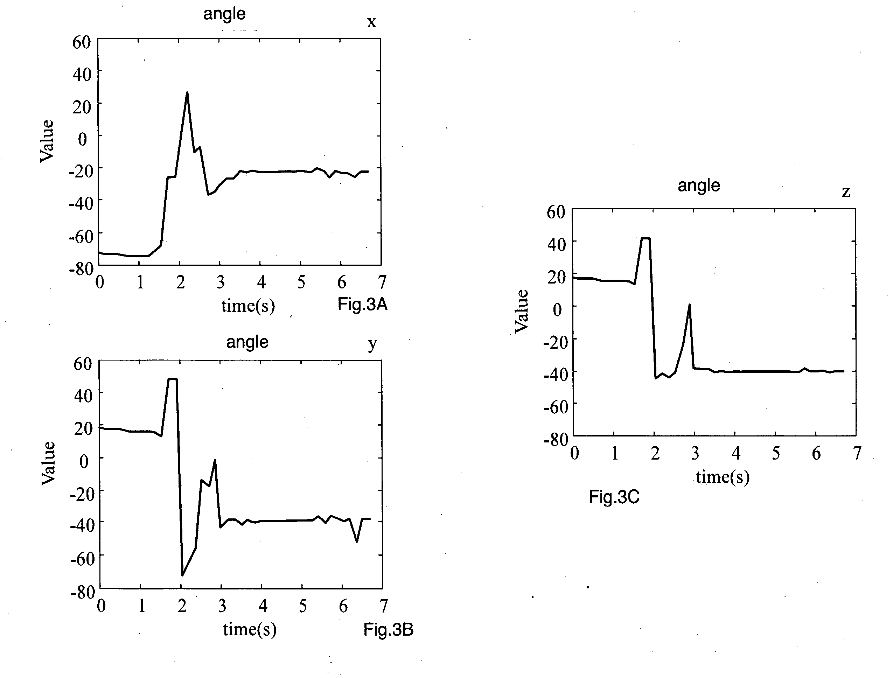

Please refer to Fig. 3A, Fig. 3B and Fig. 3C, because the characteristics of the fall event has a fast, rapid change in the characteristics of the identification of whether the fall or not. Set two groups of conditions, a group of time conditions, and the other group for the acceleration threshold conditions. When the processor detects that the data graph meet the two sets of conditions at the same time (the trigger time is short and the acceleration change is large), the processor acts to judge whether the fall event occurs. As the figures show, when the acceleration of the Y axis and the Z axis changes greatly in the time interval of 2-3 seconds in the time axis, the processor 113 judges that to indicate a great change of the Y axis and the Z axis and accordingly determines that a fall event occurs. In addition, the values of the X-axis, the Y-axis, and the Z-axis direction are the G-sensitivities (acceleration in degrees) in the direction, and the direction of the horizontal axis is the elapsed time value.

In addition to the above method, it is possible to determine whether the user is in a falling status by detecting the direction of the user's X-axis, Y-axis or Z-axis (for example, the Z-axis moves from the high level to the low level). However, the above method of detecting the fall is only an example of the invention of the fall detection method, and is not limited to the scope of the invention.

The processor 113 may set another time threshold as an auxiliary reference parameter to identify whether the user has failed to stand up again after landing. When the processor 113 judges that a fall event occurs, a timer function is activated, and the chronograph function is used to determine the fall whether the quiescent period after the event exceeds the value to determine whether the user is to stand up again. In the general case, when the rest period exceeds the value, the degree of the fall event is determined to be serious, and the notification server dispatches the medical staff to provide emergency assistance. In another case, when the X axis, the Y axis, the Z axis of the acceleration value has undergone a significant change, you may determine the user and then stand up again. In another preferred embodiment, it is determined whether the Z-axis parameter is to be moved from the low level to the high level when it is determined whether to stand up again. If so, the user may be determined to stand up again.

The processor 113 includes an energy saving state, and a precision detection state. In the normal state, the processor 113 adjusts the number of times of the inertia sensor 111 draws the user's motion data per unit time to increase the operation time of the wearable apparatus 110. As shown in Fig. 8, when the processor 13 detects that the fall event occurs, the processor 113 switches to the precision detection state and increases the number of times the user's motion data is drawn per unit time to obtain more accurate data to more accurately determine the severity of the user's injury.

The processor 113 further detects the data of the posture of the user at the time of the stand-by after the processor 113 determines that the fall event occurs. Then, the processor 113 is configured to compare the data of the posture and the data of the normal posture in the database to determine the severity of the damage to the user. The processor 113 may include multiple pre-set abnormal posture conditions, and classify the user's motion data according to the abnormal posture condition to determine the type of injury.

The normal posture conditions described in the invention refer to conditions set by the processor 113 based on the data initially detected by the user or, in alternative configurations not falling within the scope of the claimed invention, may be based on the user's motion data obtained by the general person in general walking habits. The conditions may be data or calculus law and may not be limited in the invention. The abnormal posture conditions described in the invention refer to the calculus law set by the processor 113 based on the user's motion data obtained by the user or the general person in the abnormal posture and may not be limited in the invention. Through the fuzzy algorithm (Fuzzy Algorithm) to compare the data of posture with the conditions of the abnormal posture. The type of the user's injury is judged by analyzing the condition in which the posture data coincides with correlation in a data pattern. Some of common abnormal states are only given by way of illustrations, and the present invention is not limited to following embodiments.

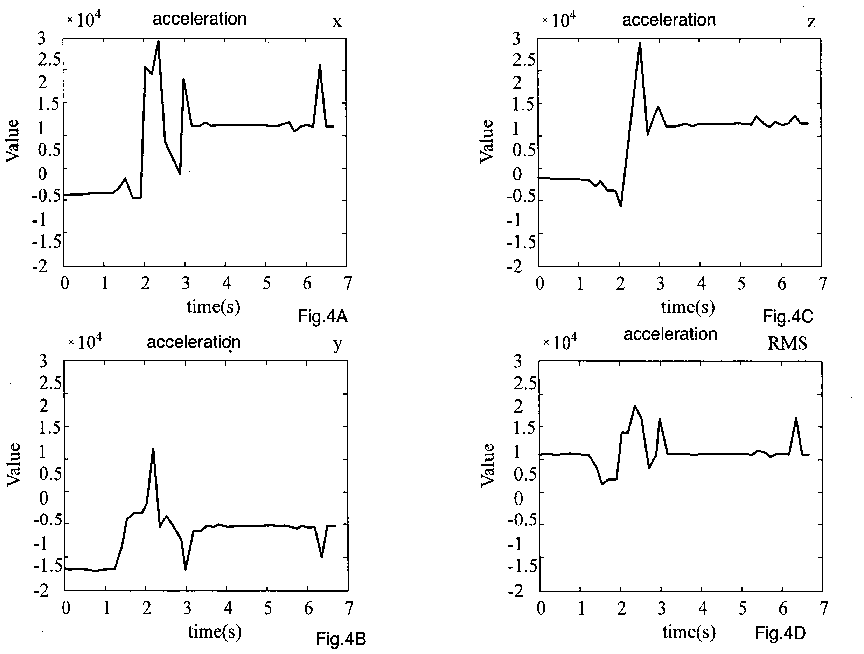

Please refer to Fig. 4A, Fig. 4B, Fig. 4C and Fig. 4Dto reveal the data when the user is moving normally. By the figure, it is known that the normal person moves normally forward, a walking mode includes the acceleration of forward, the acceleration of slightly shaking up and down during walking, and the acceleration of a body swinging left and right as moves. In Fig. 4A, Fig. 4B, Fig. 4C and Fig. 4D, the X-axis acceleration pattern swings between the positive and negative areas according to timing law.
Because of the footsteps, the side of a human body may appear tilted state, and in the exchange forward situation of left foot and right foot, the human body may naturally swings left and right. The Y-axis acceleration pattern is based on the timing of regular swing, the main reason is that the acceleration is the largest when people are moving in pace after moving forward, and decreases before the other foot touches on ground. The Z-axis acceleration pattern swings between the positive and negative areas according to timing law. The main reason is that in the road mode, step by step may produce a slight swing up and down. The processor 113 may set threshold value or store the data as the normal posture conditions, and meet the conditions of the threshold value or match the graphics to determine whether the user's posture is normal based on the normal walking data graph.

When determining the normal posture condition is not met, the user's motion data of is compared with the abnormal posture condition to determine what kind of injury the user may be. It may be noted that the invention does not necessarily need to be the order of the above comparison, may directly compare the user's motion data with abnormal posture conditions to determine the severity of the damage to the user. The default posture in the system is, for example:

### Moving slowly:

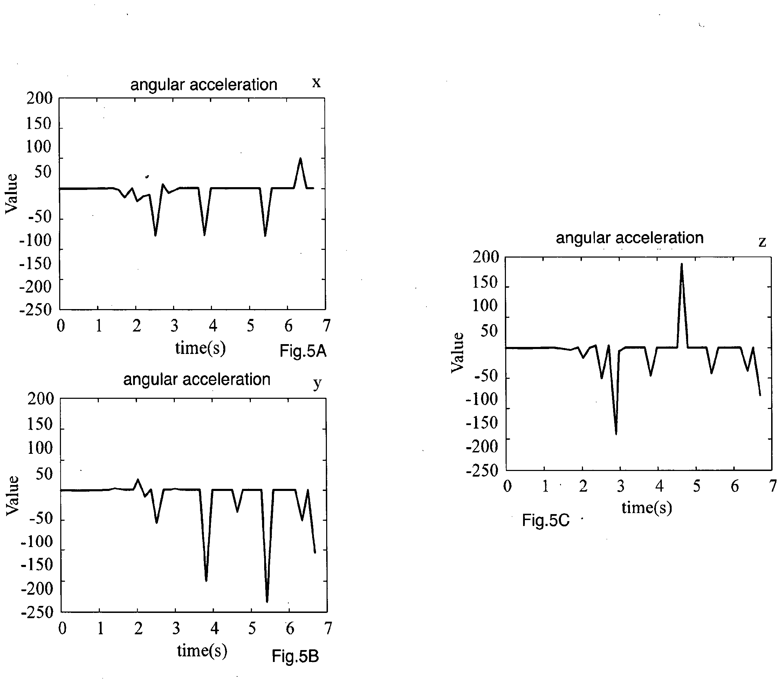

Please refer to Fig. 5A, Fig. 5B and Fig. 5C, the general user falls and is injured, the motion speed may slow down, according to the user's motion data to determine the user's motion speed. In Fig. 5A, Fig. 5B and Fig. 5C, when the user's motion speed slows down, the level of the Y-axis acceleration is significantly reduced because the walking speed slows down, decreasing the body shaking up and down. The Z-axis acceleration change may become smaller to directly determine the threshold value as the abnormal posture condition to determine the Y-axis acceleration level. When the user's motion speed is lower than the pre-set value, that is, the user is judged to be discomfort and the motion speed becomes slow.

### Unwounded:

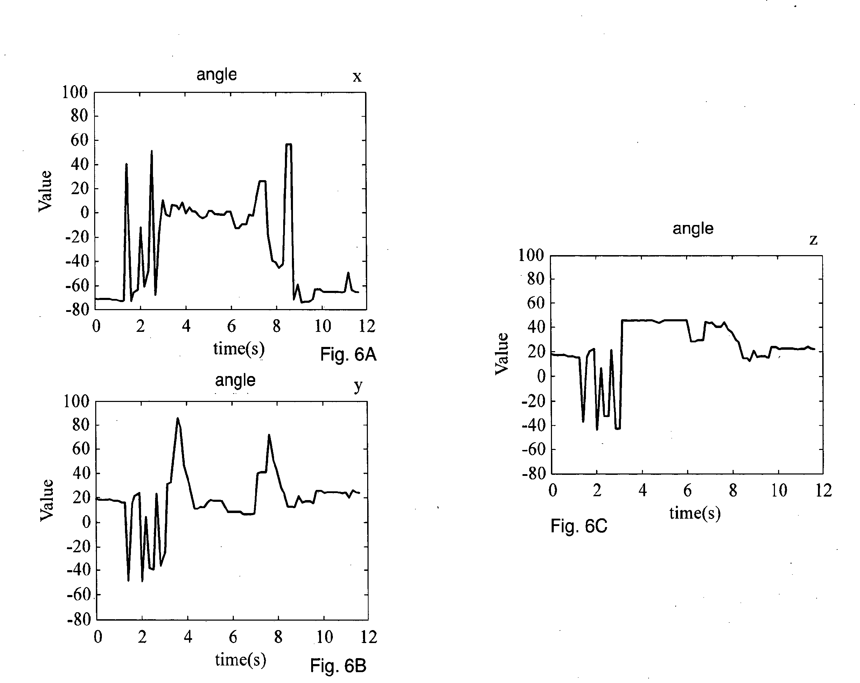

Please refer to Fig. 6A, Fig. 6B and Fig. 6C, the user falls and is injured, if the user is injured in a foot position, there may be a different situation in mobile mode and determine whether the user is injured or not according to the situation. When the user is injured in the foot, the most likely direct response to the location is the user's balance. Due to the lack of balance of the body, the user is prone to move in a side of shaking. Fig. 6A, Fig. 6B and Fig. 6Cshows the acceleration change pattern of the X-axis, Y-axis, Z-axis in left foot injury. The figure shows amount of change in the X-axis figure may be significantly shifted to the negative area because the user's left and right balance significantly tilt to one side makes the X-axis acceleration be imbalance. The acceleration change of Z axis significantly increases because shaking range up and down of the user's body increases make the acceleration change of Z-axis increase. Being capable of setting the threshold value of the X-axis changes and the threshold value of the Z-axis changes or directly storing the user's motion data as an abnormal posture condition. When meeting the threshold conditions or matching graphics, the user may be judged as the abnormal posture of foot injury and dragging.

### Beating:

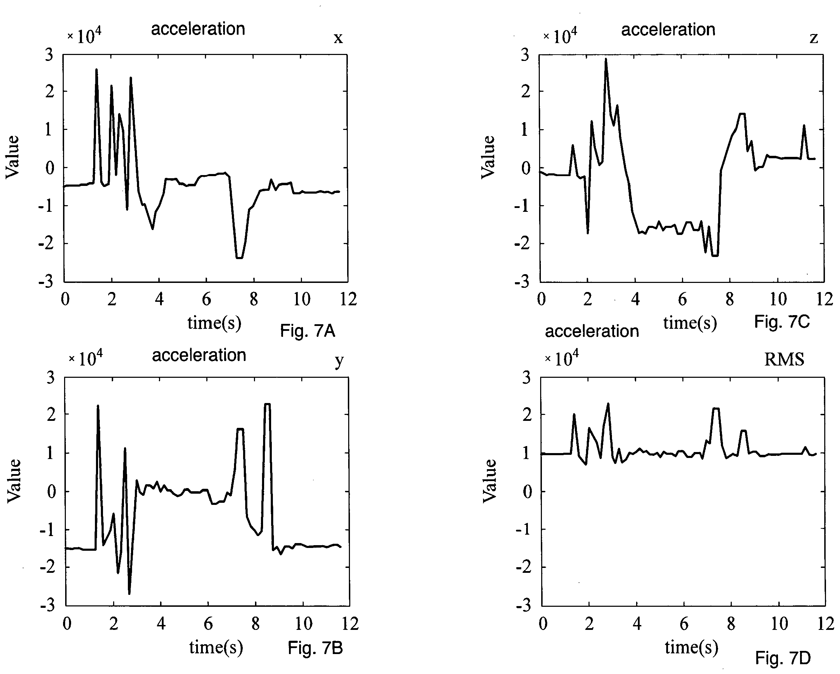

Please refer to Fig. 7A, Fig. 7B, Fig. 7C and Fig. 7D, when the user injures the foot, especially on a single foot, the user may have a beating situation. For example, when the heavy severity of single foot is injured, the injured may avoid affected area (foot) to touch ground and may have single-legged beating situation. Fig. 7A, Fig. 7B, Fig. 7C and Fig. 7D shows the situation the user's foot is injured and beats. The acceleration value of the Y-axis from the peak to the valley may increase (like: the amount of change), and the acceleration of the valley is likely to be zero to make an interval between each peak. The main reason is the pause generated between each beating. Due to the relationship between beating, the range and acceleration may increase when jump up and down and then the changes of the Z-axis may increase significantly. Beating the pause may make the interval time between wave and wave increase. Being capable of setting the threshold value or directly storing the user's motion data as an abnormal posture condition. When meeting the threshold conditions or matching graphics, the user may be judged as the abnormal posture of foot injury and beating.

By setting the above abnormal conditions, the user's injury may be classified to determine the severity of the user's injury. However, the above methods are merely an illustration of the present invention. In addition to the above methods, the static posture may be applied to the user through a magnetic sensor (M-Sensor), a tilt sensor (Tilt Sensor), or other similar apparatus to determine the degree of injury to the user. The invention does not limit the implementation of the above situations, which must first be described.

In summary, the invention may be analyzing the posture or walking mode data after the fall to determine the severity of the damage to the user, and decide whether to send emergency ambulance personnel. Comparing with the know-how technology this may accurately analyze the user's injury and thus may reduce the occurrence of misjudgements. In addition, the invention may prejudge the seriousness of the user's fall and the condition of the injury by the user's posture or walking mode. The above data may be prepared in advance to prepare the corresponding equipment or the corresponding medical personnel to the scene.

Fig. 8A, Fig. 8B, Fig. 8C, Fig. 9A, Fig. 9B, Fig 9C, Fig. 10A, Fig. 10B, Fig. 10C, Fig.100, Fig. 11A, Fig. 11B, Fig. 11C, Fig. 12A, Fig. 12B, Fig. 12C, Fig. 13A, Fig. 13B,Fig.13C, Fig. 13D, Fig. 14A, Fig. 14B, Fig. 14C, Fig. 15A, Fig. 15B, Fig. 15C, Fig.16A,Fig. 16B, Fig. 16C, Fig. 16D, Fig. 17A, Fig. 17B, Fig. 17C, Fig. 18A, Fig. 18B, Fig.18C, Fig. 19A, Fig. 19B, Fig. 19C, Fig. 19D, Fig. 20A, Fig. 20B, Fig. 20C, Fig.21A, Fig.21B, Fig. 21C, Fig. 22A, Fig. 22B, Fig. 22C, Fig. 22D, Fig. 23A, Fig. 23B,Fig. 23C, Fig.24A, Fig. 24B, Fig. 24C, Fig. 25A, Fig. 25B, Fig. 25C, Fig. 25D, Fig.26A, Fig. 26B, Fig.26C, Fig. 27A, Fig. 27B, Fig. 27C, Fig. 28A, Fig. 28B, Fig. 28C, Fig. 28D, Fig. 29A, Fig.29B, Fig. 29C, Fig. 30A, Fig. 30B, Fig. 30C, Fig. 31A, Fig. 31B, Fig. 31C, Fig. 31D, Fig.32A, Fig. 32B and Fig. 32C illustrate other experiment data. We make a lot of experiments and prove that the concept is effective.

The above description has been made in detail, but only is not intended to limit the scope of the present which is defined by the appended claims.

## Claims

1. A wearing apparatus (110) for wearing by a user, the wearing apparatus (110) comprising:
an inertial sensor (111), wherein the inertial sensor (111) is based on the user's motion to obtain user's motion data;
a communication module (112), wherein the communication module (112) is used to link and transmit data to a server for real-time monitoring through the server; and
a processor (113), wherein the processor (113) is connected with the inertial sensor (111) and the communication module (112), and wherein, the processor (113) is further configured to acquire the user's motion data when the user is to stand up again via the inertial sensor (111) after the processor (113) detects user's fallback state based on the user's motion data, matches the user's motion data according to normal posture conditions and / or abnormal posture conditions in a database (114, 121) to report severity of the user's injury to a dispenser via the communication module (112); **characterized in that** the processor (113) is configured to increase the number of times of the inertia sensor drawing the user's motion data per unit time to obtain more accurate data when the processor (113) detects user's fall state;
and **in that** the processor (113) is further configured to preliminary record the user's motion data obtained by the user or the normal person's normal posture to establish the normal posture condition.

2. The wearing apparatus (110) of claim 1, wherein the processor (113) is configured to classify the user's motion data according to a plurality of pre-set abnormal posture conditions to determine a type of injury of the user.

3. The wearing apparatus (110) of claim 2, wherein the processor (113) is configured to be connected to a warning apparatus (125) and to generate and send a warning signal to the warning apparatus (125) based on the type of injury detected by the processor (113).

## Patentansprüche

1. Tragevorrichtung (110) zum Tragen durch einen Benutzer, wobei die Tragevorrichtung (110) umfasst:
einen Trägheitssensor (111), wobei der Trägheitssensor (111) auf der Bewegung des Benutzers basiert, um die Bewegungsdaten des Benutzers zu erhalten;
ein Kommunikationsmodul (112), wobei das Kommunikationsmodul (112) zur Verbindung und Übertragung von Daten an einen Server zur Echtzeitüberwachung durch den Server verwendet wird; und
einen Prozessor (113), wobei der Prozessor (113) mit dem Trägheitssensor (111) und dem Kommunikationsmodul (112) verbunden ist, und wobei der Prozessor (113) ferner so konfiguriert ist, dass er die Bewegungsdaten des Benutzers erfasst, wenn der Benutzer unter Verwendung des Trägheitssensors (111) wieder aufstehen soll, nachdem der Prozessor (113) den Rückfallzustand des Benutzers auf der Grundlage der Bewegungsdaten des Benutzers erfasst hat, die Bewegungsdaten des Benutzers entsprechend normaler Haltungsbedingungen und/oder abnormaler Haltungsbedingungen in einer Datenbank (114, 121) abgleicht, um die Schwere der Verletzung des Benutzers über das Kommunikationsmodul (112) an einen Spender zu melden;
**dadurch gekennzeichnet, dass**
der Prozessor (113) so konfiguriert ist, dass er die Anzahl der Male, die der Trägheitssensor die Bewegungsdaten des Benutzers pro Zeiteinheit aufnimmt, erhöht, um genauere Daten zu erhalten, wenn der Prozessor (113) den Sturzzustand des Benutzers feststellt; und dass der Prozessor (113) ferner so konfiguriert ist, dass er die vom Benutzer erhaltenen Bewegungsdaten des Benutzers oder die normale Körperhaltung der normalen Person vorläufig aufzeichnet, um den Zustand der normalen Körperhaltung festzustellen.

2. Tragevorrichtung (110) nach Anspruch 1, wobei der Prozessor (113) so konfiguriert ist, dass er die Bewegungsdaten des Benutzers gemäß einer Vielzahl von voreingestellten abnormalen Haltungsbedingungen klassifiziert, um eine Art von Verletzung des Benutzers zu bestimmen.

3. Tragevorrichtung (110) nach Anspruch 2, wobei der Prozessor (113) so konfiguriert ist, dass er mit einer Warnvorrichtung (125) verbunden ist und auf der Grundlage der vom Prozessor (113) erfassten Art der Verletzung ein Warnsignal erzeugt und an die Warnvorrichtung (125) sendet.

## Revendications

1. Appareil de port (110) destiné à être porté par un utilisateur, l'appareil de port (110) comprenant :
un capteur inertiel (111), dans lequel le capteur inertiel (111) est basé sur le mouvement de l'utilisateur pour obtenir des données sur le mouvement de l'utilisateur ;
un module de communication (112), dans lequel le module de communication (112) est utilisé pour relier et transmettre des données à un serveur en vue d'une surveillance en temps réel par l'intermédiaire du serveur ; et
un processeur (113), dans lequel le processeur (113) est connecté au capteur inertiel (111) et au module de communication (112), et dans lequel, le processeur (113) est en outre configuré pour acquérir les données de mouvement de l'utilisateur lorsque l'utilisateur doit se relever via le capteur inertiel (111 ) après que le processeur (113) ait détecté l'état de repli de l'utilisateur sur la base des données de mouvement de l'utilisateur, fait correspondre les données de mouvement de l'utilisateur en fonction des conditions de posture normales et/ou anormales dans une base de données (114, 121) pour signaler la gravité de la blessure de l'utilisateur à un distributeur par l'intermédiaire du module de communication (112) ; **caractérisé par le fait que** le processeur (113) est configuré pour augmenter le nombre de fois que le capteur d'inertie dessine les données de mouvement de l'utilisateur par unité de temps afin d'obtenir des données plus précises lorsque le processeur (113) détecte l'état de chute de l'utilisateur ; et **par le fait que** le processeur (113) est en outre configuré pour enregistrer au préalable les données de mouvement de l'utilisateur obtenues par l'utilisateur ou la posture normale d'une personne normale afin d'établir la condition de posture normale.

2. L'appareil de port (110) de la revendication 1, dans lequel le processeur (113) est configuré pour classer les données de mouvement de l'utilisateur en fonction d'une pluralité de conditions de posture anormale prédéfinies afin de déterminer un type de blessure de l'utilisateur.

3. L'appareil de port (110) de la revendication 2, dans lequel le processeur (113) est configuré pour être connecté à un appareil d'avertissement (125) et pour générer et envoyer un signal d'avertissement à l'appareil d'avertissement (125) sur la base du type de blessure détecté par le processeur (113).
